Europäisches Patentamt

European Patent Office

Office européen des brevets

(11) Publication number: **0 316 449 B1**

(19)

# EUROPEAN PATENT SPECIFICATION

(12)

(45) Date of publication of patent specification: **08.12.93**    (51) Int. Cl.5: **C12M 3/00**, C12N 5/00,
//C12M1/06

(21) Application number: **87904965.8**

(22) Date of filing: **31.07.87**

(86) International application number:
**PCT/JP87/00573**

(87) International publication number:
**WO 88/00965 (11.02.88 88/04)**

(54) **METHOD AND APPARATUS FOR INCUBATING ANIMAL CELLS.**

(30) Priority: **08.08.86 JP 185131/86**

(43) Date of publication of application:
**24.05.89 Bulletin 89/21**

(45) Publication of the grant of the patent:
**08.12.93 Bulletin 93/49**

(84) Designated Contracting States:
**CH DE FR GB LI SE**

(56) References cited:
**DE-C- 911 962**
**JP-U-57 164 600**
**JP-Y- 4 419 179**

**CANADIAN JOURNAL OF CHEMICAL ENGI-
NEERING, vol. 64, no. 4, 1986; S.R. ADAMSON
et al., pp. 531-539&NUM;**

(73) Proprietor: **HITACHI, LTD.**
**6, Kanda Surugadai 4-chome**
**Chiyoda-ku, Tokyo 101(JP)**

(72) Inventor: **ISHIBASHI, Tadashi**
**23-22, Ohse-cho 3-chome**
**Hitachi-shi Ibaraki 317(JP)**
Inventor: **KAWAGUCHI, Hideo**
**301, 36-2, Nishinarusawa-cho 1-chome**
**Hitachi-shi Ibaraki 316(JP)**
Inventor: **YOSHINO, Saeko**
**304, 6-6, Ohmika-cho 6-chome**
**Hitachi-shi Ibaraki 317(JP)**
Inventor: **ODAWARA, Yoji**
**21-6, Nishinarusawa-cho 4-chome**
**Hitachi-shi Ibaraki 316(JP)**

(74) Representative: **Strehl Schübel-Hopf Groening
& Partner**
**Maximilianstrasse 54**
**D-80538 München (DE)**

Rank Xerox (UK) Business Services
(3.10/3.6/3.3.1)

## Description

The present invention relates to a method and apparatus for culturing animal cells in a mechanical stirring culture tank according to the preambles of claims 1 and 9.

When multiplying animal cells in a mechanical stirring culture tank, oxygen existing in a vapor phase at an upper portion of the tank is dissolved in a liquid phase through the liquid surface, thereby feeding necessary oxygen. A migration rate (dissolution rate from vapor phase to liquid phase) of oxygen in this case is considerably affected by the number of rotations of a stirrer (blade) in conjunction with a vapor-liquid interface area per volume of culture solution and renewal of the vapor-liquid interface. However, an increase in the number of rotations leads to an increase in shearing force, which may hurt or kill the cell, and hence there is a limit. Further, since a serum is added to the culture solution for culturing animal cells, it foams violently from aerating agitation; therefore, a ratio of liquid quantity to tank capacity must be minimized. Further, the culture solution foams so as to overflow, and thus the culturing cannot be kept going. Consequently, a transfer of oxygen (dissolution in liquid phase from vapor phase) cannot be accelerated by a large-volume aeration and strong agitation as in the case of a microbial culture; therefore, various improvements have been attempted.

As an attempt for the improvement, ① oxygen-containing gas will be aerated in the culture solution to increase the gas-liquid interface area, or ② the liquid level will be agitated (disturbed) to accelerate a renewal of the gas-liquid interface. Details are given in Bibliography 1: [Ann. New York Acad. Sci. Vol. 413Pp. 361 - 364 (1983)] for ① and in Bibliography 2: Biotechnology and Bioengineering, Vol. XXVIII, Pp. 122 to 125 (1985) for ②. However, a satisfactory method and apparatus have not yet been developed.

The aforementioned defects are unavoidable with the prior art, and an improvement has still been attempted presently, however, the problems will be taken up below in detail.

(1) Aerating agitation system:

A oxygen-containing gas is blown (aerated) into culture solution, the solution is further agitated by a stirrer blade and the gas is dispersed in the solution. A benefit of the method is that the gas forms small bubbles and resides in the solution and, therefore, a gas-liquid interface area increases, and a transfer of oxygen is accelerated. However, a protein component such as serum or the like is often contained in the culture solution used for culturing animal cells and, therefore, it very readily foams. Further, the foaming will decompose a cell membrane (so mentioned in Bibliography 1), thus inhibiting a cell propagation. As a counter-measure thereto, it is conceivable that a defoaming agent (or surface active agent) may be added, however, unlike a microbial cell, an animal cell is weak in resistance against the surface active agent, and thus the propagation will be prevented thereby, and hence it cannot be used. Nothing has yet been found which is less virulent and efficacious.

On the other hand, there is an approach in which the cell is included in a semipermeable capsule such as sodium alginate or the like so as to protect the cell from being harmed by foaming, the capsule is suspended in a culture medium, and an oxygen-containing gas is blown thereinto to culture it in a foamed state. However, the system requires an apparatus capable of preparing the capsule in sterility, thus complicating the culture system. Further, since the culture medium comes into an exhaust system because of foaming, a sterilizing filter must be prevented from clogging and a piping must be cleaned accordingly.

(2) Liquid level stirring system:

The method comprises agitating a liquid level with a blade provided on a stirring shaft for disturbing the liquid level, thereby increasing a gas-liquid contact area. The blade, called a surface aerator, is effective in its own way at a low-speed rotation; however, it functions like a foaming machine when operated at a high-speed rotation to obtain a high oxygen transfer rate. Consequently, there is an attempt being made at improving the oxygen transfer rate by injecting an oxygen gas into a gas phase.

As described above, a relation between an operating condition and a foaming feature of the culture solution has not been taken fully into consideration in the prior art, which is one of the influential factors to limit a realization of high density culture.

DE-C-911 962 is related to an apparatus wherein blow nozzles for blowing oxygen-containing gas into a fermentating solution are provided below the liquid level.

An article "Cultivation of Anchorage-dependent Cells and Their Applications" by A.L. van Wezel in J. Chem. Techn. Biotechnol. 1982, 32, 318-323 discloses a method of blowing oxygen-containing gas over the surface of a culture fluid in order to improve the cell density in the solution.

It is an object of the invention to provide a method and apparatus for culturing animal cells, wherein the aeration and thus the oxygen exchange of a culture solution is further improved to thereby enable an increase in cell density and to avoid having bubbles drawn into the liquid.

This object is met by the invention as set forth in claims 1 and 9. Preferred embodiments of the invention are described in the dependent claims.

A first feature of the invention comprises a method for culturing animal cells, whereby a culture solution is stirred while suppressing a formation of turbulence or vortex of the liquid level for culturing animal cells in a mechanical stirring culture tank, an oxygen-containing gas is discharged continuously toward the liquid level from a blow nozzle to form a dimple or dimples on the liquid level. It is desirable in this case that during operation the product $f_G$ of a gas blow quantity Q (cm$^3$/s) and a gas linear velocity v (cm/s) at a nozzle tip is kept at $2.5 \times 10^5 \times h$ or smaller with a distance from the nozzle tip to the still liquid level as h cm. Accordingly, when the nozzle bore is 1 mm, it is desirable that a linear velocity of the oxygen-containing gas discharged from the nozzle vertically toward the liquid level is controlled to between 5 m/s and 65 m/s at the liquid level.

A second feature of the invention comprises an apparatus for culturing animal cells in a mechanical stirring culture tank, wherein a single or a plurality of gas blow nozzles for discharging an oxygen-containing gas continuously is provided facing toward the liquid level in a gas phase space at an upper portion of the liquid level.

In this case, it is desirable that the stirrer blade for agitating the culture solution may be structured to have a center-punched rectangular plate twisted 1/2 turn.

A third feature of the invention comprises a method for culturing animal cells in a culture tank while agitating the culture solution where animal cells are dispersed with a stirrer, wherein the culture solution is agitated while keeping the liquid level substantially smooth, an oxygen-containing gas is discharged continuously toward the liquid level from gas blow nozzles having a multiplicity of holes therefor, a multiplicity of dimples are thus formed on the liquid level, thereby dissolving the oxygen gas into the culture solution.

A fourth feature of the invention comprises a method for culturing animal cells while agitating mechanically the culture soltuion where animal cells are dispersed in a culture tank, wherein the culture solution is agitated while preventing foaming of the culture solution and rupture of the animal cells, an oxygen-containing gas is discharged toward the liquid level from gas blow nozzles, dimples are thus formed on the liquid level, thereby dissolving the oxygen gas into the culture solution.

A fifth feature of the invention comprises a method for culturing animal cells in a mechanical stirring culture tank, wherein the culture solution is agitated while suppressing a formation of turbulence or vortex of the liquid level, an oxygen-containing gas is discharged continuously toward the liquid level from gas blow nozzles, dimples are thus formed on the liquid level and a flow of the culture solution from a bottom of the dimple toward the liquid level is formed, thereby dissolving the oxygen gas into the culture solution.

A sixth feature of the invention comprises an apparatus for culturing animal cells having a culture tank containing a culture soltuion therein where animal cells are dispersed, a stirrer for agitating the culture solution and a means for feeding an oxygen gas to the liquid level of the culture solution, wherein gas blow nozzles for discharging the oxygen-containing gas continuously toward the liquid level are provided in a gas phase space at an upper portion of the liquid level.

A seventh feature of the invention comprises an apparatus for culturing animal cells having a culture tank containing a culture solution therein, where animal cells are dispersed, a stirrer for agitating the culture solution and a means for feeding an oxygen gas to the liquid level of the culture solution, wherein gas blow nozzles for discharging the oxygen gas almost vertically toward the liquid level are provided in a gas phase space at an upper portion of the liquid level.

An eighth feature of the invention comprises an apparatus for culturing animal cells having a culture tank containing a culture solution therein, where animal cells are dispersed, a stirrer for agitating the culture solution and a means for feeding an oxygen gas to the liquid level of the culture solution, which is provided with gas blow nozzles for discharging the oxygen-containing gas almost vertically toward the liquid level, a control means for controlling a discharge pressure from the nozzle, thereby forming dimples on the liquid level.

A ninth feature of the invention comprises a method for culturing animal cells dispersed in a culture solution in a culture tank while agitating mechanically, wherein the culture solution is agitated while preventing foaming of the culture solution and rupture of the animal cells, an oxygen-containing gas is discharged from gas blow nozzles vertically toward the liquid level at a rate of 5m/s to 65m/s (a linear velocity at the liquid level when calculated with a nozzle bore as 1 mm), dimples are thus formed on the liquid level, thereby dissolving the oxygen gas into the culture solution.

The invention will now be described in detail with reference to the accompanying drawings.

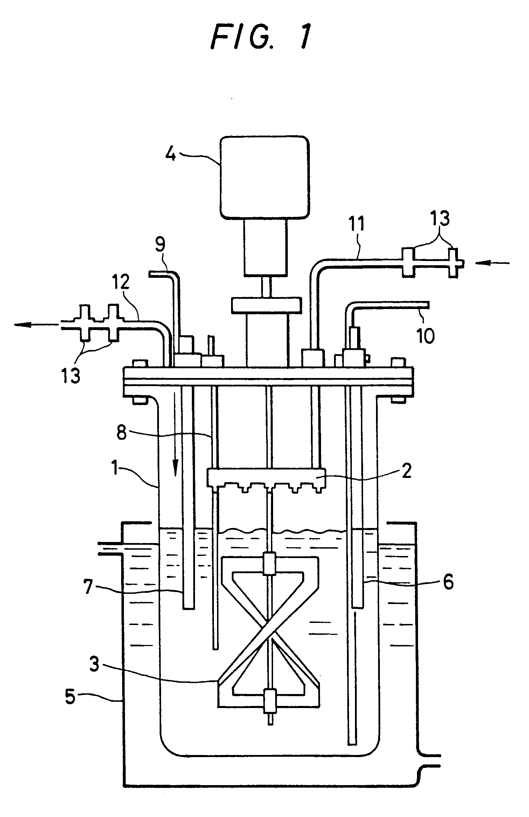

In Fig. 1, a culture tank 1 is a mechanical stirring type in which a culture solution is agitated by a stirring blade 3. The stirring blade 3 is fitted to a driving shaft of a stirrer motor 4 and thus is rotated by the stirrer motor 4. The stirring blade 3 may be rotated under a magnet system, which is not particularly limited. There provided in the tank are a gas blow pipe 2 for discharging an oxygen-containing gas onto the liquid level, a pH sensor 6, a dissolved oxygen sensor 7 and a temperature sensor 8. Then, a thermostatic vessel 5 heats the culture tank 1. A sterilizing filter 13 mounted on an air inlet 11 and an exhaust port 12 has a bore of 0.2 to 0.45 $\mu$m, removes germs in the oxygen-containing gas and also prevents germs from coming in through the exhaust port 2. Then, to suppress evaporation of the culture solution accompanying exhaust, the oxygen-containing gas for which a steam pressure is adjusted to a value approximate to a saturation steam pressure of the culture temperature is fed to the culture tank 1. Thus the filter uses a hydrophobic material such as Teflon or the like less susceptible to clogging with dew.

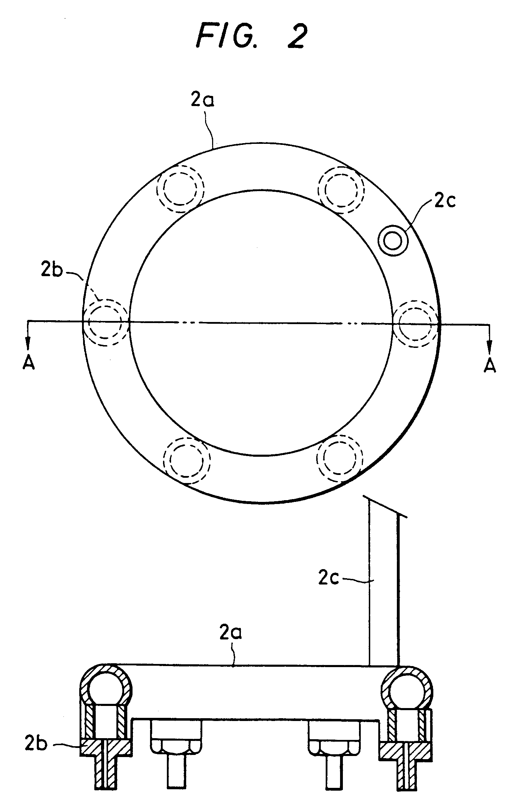

Fig. 2 represents one example of the gas blow pipe 2. The gas blow pipe 2 is constituted of a buffer pipe 2a, a blow nozzle 2b and an air pipe 2c. The oxygen-containing gas comes into the buffer pipe 2a by way of the air pipe 2c and is discharged continuously under an equalized pressure from each blast nozzle 2b mounted on a lower portion of the buffer pipe 2a. A gas discharge face of the blow nozzle 2b is directed to the liquid level, however, it may preferably be kept parallel with a still liquid level. Further, a bore of the blow nozzle 2b and a distance between the gas discharge face and the liquid level will be determined so that the discharged oxygen-containing gas may form an inverted conical dimple on the liquid level. When the nozzle bore is 1 mm, a linear velocity of the oxygen-containing gas in the vertical direction at the liquid level will be adjusted to 5 m/s or over, thereby forming the inverted conical dimple on the liquid level. The number of blow nozzles 2b is not particularly limited, which may be set so as to obtain a maximum oxygen transfer rate. If a plurality of blow nozzles 2b are installed, they will be located in a plane so as not to allow the dimples to overlap each other on the liquid level.

Meanwhile, when a great deal of turbulence is present on the liquid surface, gas is drawn into the solution by the oxygen-containing gas discharged from the blow nozzle 2b, thus causing a foaming.

Then, when a deep vortex is formed on the liquid surface, the blow nozzle 2b is distant from the liquid level, and thus a dimple is not formed on the liquid level. Accordingly, a liquid level plane with less turbulence will be preferable.

A formation of turbulence or vortex on the liquid level is influenced chiefly by a shape of the stirring blade 3, therefore a proper one must be selected. The center-punched rectangular stirring blade 3 shown in Fig. 1 exemplifies what is capable of suppressing formation of turbulence or vortex on the liquid level.

An operation for culturing animal cells on the apparatus will be described with reference to Fig. 1.

The animal cell may be classified roughly into that of growing on a solid surface and that which can be propagated in a suspended state. In the former, it can be cultured according to a microcarrier process, namely a process wherein the cell is attached to a bead such as dextran or the like, and the bead is suspended. Accordingly, the present invention relating to a suspension culture can be used to the animal cells of both the aforementioned kinds.

A culture medium is fed into the culture tank 1 in sterility from a medium feed port 9. The culture medium can be fed leaving a minimum space (including a distance from the liquid level to the nozzle discharge face) necessary for installing the gas blow pipe 2, and a preferable rate of the working volume to the tank volume will be 40 to 70%. Culturing starts upon inoculation of a seed cell from the medium feed port 9. A temperature controller operates with a signal of the temperature sensor 8, and thus temperature and flow rate of hot water to be fed to the thermostatic tank 5 are controlled, thereby controlling temperature of the culture solution to within a predetermined range. At the beginning of culture an oxygen-containing gas including 5% or so of carbon dioxide is fed to a gas phase portion of the culture tank 1 from the gas blow pipe 2 at such a flow rate as not form a dimple on the liquid level. Oxygen necessary for the cell propagation is fed from the gas phase through the liquid level. If the cell density is low, then the dissolved oxygen concentration is not at a level to be a limiting factor for the cell propagation. The stirring blade 3 may run moderately fast enough to disperse the cells uniformly. Then, whenever oxygen consumption increases as the cells are propagated and the dissolved oxygen concentration drops to a level to be a limiting factor for the cell propagation, a dimple formation on the liquid level is commenced by blowing oxygen containing gas. That is, a regulating valve for flow rate of the oxygen-containing gas is opened by a dissolved oxygen controller having received a signal from the oxygen sensor 7, and the flow rate is increased so as to have a linear velocity in the vertical direction at the liquid surface of 5 m/s or more. A timing for the increase will be taken at the time when a level of the dissolved oxygen concentration measured every 10 to 30 minutes is at a level to be a limiting factor or below. Further, the dissolved oxygen concentration of a higher level inhibits the cell propagation. Accordingly, the flow rate will be controlled so as to provide a dissolved oxygen concentration which is between a value working as a limiting factor for the cell propagation and a value causing an impediment to the cell propagation. By controlling the dissolved oxygen concentration as above within the aforementioned range, as the cell density increases, the flow rate of oxygen-containing gas will gradually increase. However, when the product $f_G$ of the quantity of gas blown into the gas blow pipe 2 and the gas linear velocity at a nozzle tip becomes greater than $2.5 \times 10^5 \times h$, a vertical linear velocity at the liquid level of the oxygen-containing gas discharged from the blow nozzle 2b of the gas blow pipe 2 becomes greater than 65 m/s when the nozzle bore is 1 mm, and bubbles are drawn into the liquid, thus causing a foaming of the culture solution. That is, there is a limit to the flow gas quantity. Accordingly, when the flow rate of the oxygen-containing gas becomes greater than a threshold, oxygen partial pressure of the oxygen-containing gas should be raised.

Meanwhile, when the cell density is as high at $1 \times 10^6$ cells/m$\ell$ or over, wastes are accumulated, and a nutritive source becomes insufficient. Thus the culture solution is extracted from a culture solution outlet 10, and the cells are separated in sterility. Then, the cells are returned to the culture tank 1 from the culture medium feed port 9 together with a new culture medium. The operation is carried out either intermittently or continuously. Further, a separating method for the cells is not particularly limited. However, the pH-value becomes less than an optimum value through this operation only. The reason is that quantity and rate of a carbon dioxide generation are increased with an increase of the cell density, and thus the dissolved carbon dioxide increases. According to the invention, a liquid phase and a gas phase of the carbon dioxide are exchanged in good condition. Accordingly, a mixing ratio of the oxygen-containing gas to the carbon dioxide gas is changed by a pH meter having received a signal of the pH sensor 6. That is, the carbon dioxide of liquid phase is discharged to gas phase to increase a pH-value by blowing in the oxygen-containing gas while reducing the mixing ratio of carbon dioxide gas when the pH-value goes lower than the optimum value. In this case, the mixing ratio of the carbon dioxide gas will be reduced gradually at the ratio of 0.1 to 0.5%. Further, when lactic acid is accumulated so much, an addition of alkali agent such as sodium bicarbonate or the like will be effective.

Since an operation wherein an oxygen-containing gas is discharged continuously toward a liquid level from a nozzle, and thus an inverted conical dimple is formed on the liquid level, increases a gas liquid interface area due to the dimple, and also produces forcedly a flow running from a bottom of the inverted conical dimple toward the liquid level, a renewal rate of the gas-liquid interface is enhanced, thereby accelerating the oxygen transfer rate. In this case, if the kinetic energy of the oxygen-containing gas discharged from the nozzle at the liquid surface is controlled to such a degree as will depress the liquid surface, then the oxygen-containing gas will never force into the liquid phase by overcoming a tension of the liquid surface. That is, a foaming of the culture solution can be suppressed.

According to the function described as above, a foaming of the culture solution will be suppressed, while an oxygen transfer rate can be accelerated.

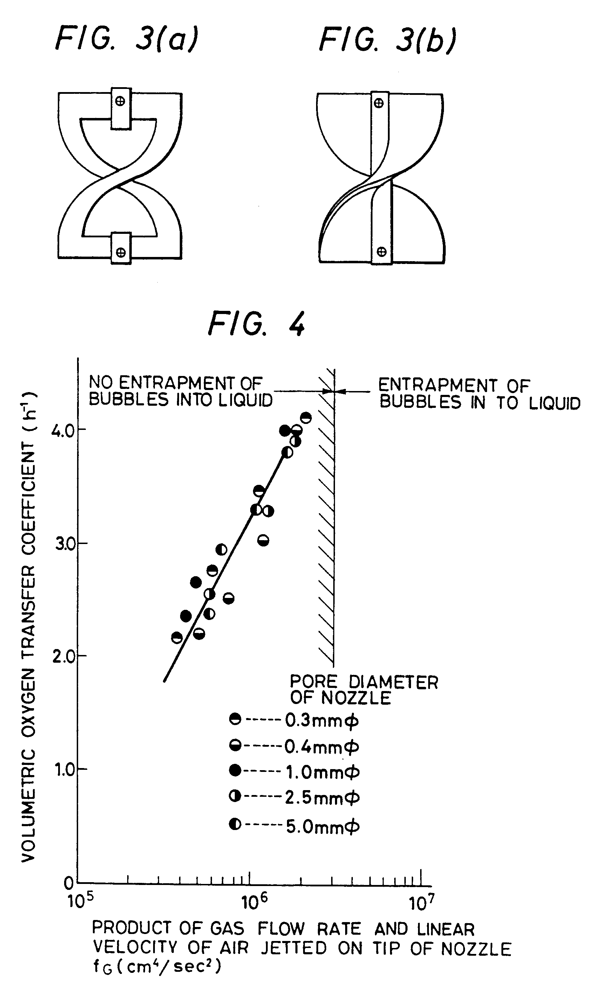

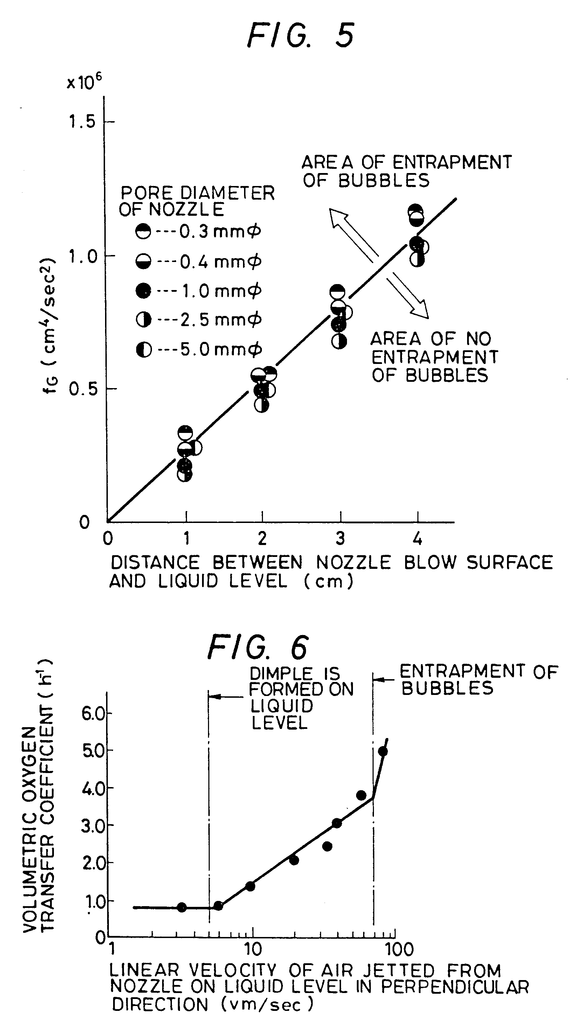

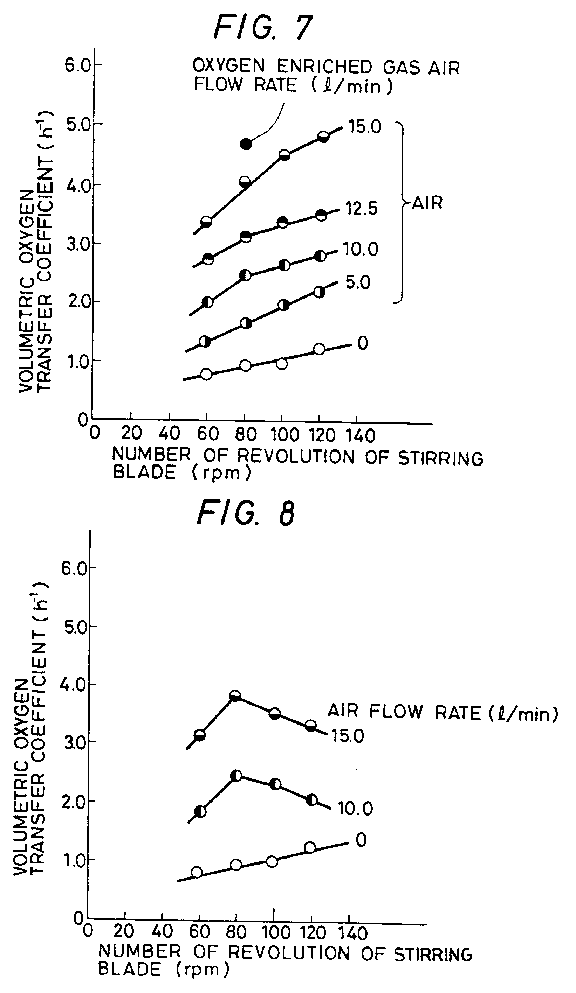

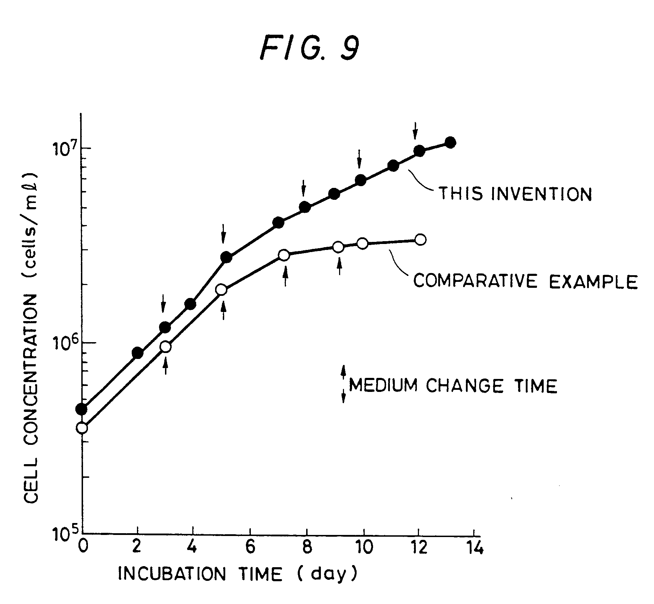

Fig. 1 is a tank structural drawing of one embodiment of the invention; Fig. 2 is a structural drawing of a gas blow pipe provided in the tank; Fig. 3 is a structural drawing of a stirring blade provided in the tank; Fig. 4 is a diagram indicating a critical value whereat gas is drawn into solution; Fig. 5 is a diagram indicating a relation between a value $f_G$ and a distance between a nozzle bore face and a liquid level when gas is about to be drawn into the solution; Fig. 6 is a diagram indicating a relation between a vertical linear velocity of the air discharged from a nozzle of the gas flow pipe at the liquid level and an volumetric oxygen transfer coefficient; Fig. 7 and Fig. 8 are diagrams indicating a relation between the number of rotations of the stirring blade with a air flow rate used as a parameter and an volumetric oxygen transfer rate coefficient; Fig. 9, Fig. 10 and Fig. 11 are diagrams indicating a change of cell density, dissolved oxygen concentration and pH, respectively, with a change in incubation time as the result of a culture test.

Example 1

A volumetric oxygen transfer coefficient in a water system was obtained through the gas-out method, using a dissolved oxygen meter in a culture tank provided with a gas blowing mechanism for oxygen-containing gas according to the invention.

The culture tank is structured as shown in Fig. 1, and a content volume was 5$\ell$. A tank body was made of pyrex glass and was 160 mm in outside diameter and 270 mm in height. A gas blow pipe was structured such that 14$\emptyset$ pipes with 6 blow nozzles of 1 mm in bore each mounted thereon at intervals of 60° were fitted on the lower portion of a toroidal buffer pipe 78 mm in outside diameter with the nozzle bore faces kept horizontal. Then, a stirring blade was prepared in two kinds as shown in Fig. 3. A stirring blade A had a structure for which a plate 80 mm wide and 100 mm high was center-punched to form a rectangular plate 50 mm wide and 60 mm high and was twisted 1/2 turn. A stirring blade B had a structure for which a rectangular plate 80 mm wide and 100 mm high was twisted 1/2 turn. Each of blades was fitted on a stirring shaft so that the bottom portion was at a position 20 mm from a tank bottom portion. Then, the gas blow pipe and the stirring vane were provided using as material SUS316.

Distilled water of 2.5ℓ was charged in the culture tank of the aforementioned structure, and the gas blow pipe was fitted so that each nozzle bore face was 20 mm distant from the water level. First, a nitrogen gas was aerated to lower a dissolved oxygen concentration to zero or around, a gas phase part of the tank was replaced by air, and then stirring and discharging of the air from the nozzle were started under predetermined conditions. An increase in the dissolved oxygen density with increasing time was obtained on a dissolved oxygen meter, and a volumetric oxygen transfer coefficient was obtained through the data. Then, the measuring temperature was 37 ± 1°C, and air the pressure of which had been adjusted beforehand to a saturation steam pressure of the aforementioned temperature, was used for the blow air.

Fig. 6 indicates a vertical linear velocity at the liquid level of the air discharged from the nozzle in each flow rate and a volumetric oxygen transfer coefficient. The stirring blade A was used, and the number of rotations was kept constant at 80 rpm. Further, an aeration rate of the air was kept from 0 to 22ℓ/min. The vertical linear velocity at the liquid level of the air discharged from the nozzle used a value obtained by measuring a velocity of flow at a point 20 mm away from the nozzle discharge face with a anemometer in an empty tank. A dimple on the liquid level was formed at a linear velocity of 5 m/s or over and a volumetric oxygen transfer coefficient increased concurrently therewith. However, a volumetric oxygen transfer coefficient where a linear velocity is 5 m/s or below was a value obtainable through a conventional process, namely a case where oxygen transfer from a gas phase part at an upper portion of the tank through the liquid level. Further, in the case of nozzle bore of 1 mm a volumetric oxygen transfer coefficient drastically increased at a linear velocity of 65 m/s or over, because bubbles were drawn into the liquid. When carrying out a foaming test by mixing a 10% serum therein, it was found that a foaming was generated strikingly at a linear velocity of 65 m/s or over.

Next, to examine effects by different stirring blades, volumetric oxygen transfer coefficients were measured for the stirring vanes A and B shown in Fig. 3. Fig. 7 represents a case of the stirring blade A, while Fig. 8 represents a case of the stirring blade B. The volumetric oxygen transfer coefficient increased in proportion to the number of rotations of the stirring blade in the former, however, it attained a maximum at 80 rpm and decreased thereafter in the latter. The difference was found to result from the fact that while the liquid level was even with small turbulence regardless of increase in the number of rotations of stirring in the former, a vortex was formed in the latter. This was due to the result that a linear velocity of the air at the liquid level decreased because the nozzle blow face was kept further away from the liquid level by the vortex. It was found consequently that a stirring blade had to be selected that did not produce a vortex as a result of rotations.

Meanwhile, an oxygen-rich gas may be utilized for enchancing an oxygen transfer rate. Now, therefore, the volumetric oxygen transfer coefficient was measured by means of an oxygen-rich gas with 40% oxygen. As measuring conditions, the stirring blade A was employed, and the number of rotations was kept constant at 80 rpm. Further, an aeration rate of the oxygen-rich gas was 12.5 ℓ/m.

The measurement result is shown in Fig. 5. It was found that it provided 1.5 times enhancement or so as compared with the case which air was used. A higher volumetric oxygen transfer coefficient can be obtained effectively from a combination of the invention and the oxygen-rich gas.

Example 2:

A volumetric oxygen transfer coefficient was obtained under each nozzle bore of 0.3, 0.4, 1.0, 2.5 or 5 mm in the apparatus used in Example 1.

Measuring conditions are as follows. Six nozzles were fitted on the gas blow pipe with the distance between the bore face and the liquid level being 20 mm. Distilled water of 2.5ℓ was charged in the tank, and the number of rotations was kept constant at 80 rpm by means of the stirring blade A. Then, measuring temperature was 37 ± 1°C, and the measurement was carried out according to the gas-out method as in the case of Example 1.

The measurement result is shown in Fig. 4. In the drawing, $f_G$ represents the product of a quantity of the gas blown into the gas blow pipe and a gas linear velocity at the nozzle tip.

A force F working on a flat plate where a jet flow collides with the flat plate is given by Eq. (1) in "Guide to Mechanical Designing" (issued by Maruzen Co., Ltd. on June 25, 1958), Pp. 1504.

$$F = (\gamma/g) Qv \qquad (1)$$

where $\gamma$ denotes a weight per unit volume, g denotes a gravity acceleration, Q denotes a flow rate, and v denotes a velocity of jet flow.

It has been considered that a force of the gas jetted out of the nozzle to depress the liquid level is proportional to the product of the blow gas flow rate and the gas linear velocity at the nozzle tip from the above equation. Here, a force $F_G$ of the blow gas to depress the liquid level is obtained by calculating a blow gas flow rate g ($cm^3$/s) per nozzle and a gas linear velocity (cm/s) at the nozzle tip under each condition. That is, a force f of the gas jetted out of each nozzle to depress the liquid level v is considered as Eq. (2).

$$f = k \times q \times v \quad (2)$$

where k: proportion constant. Accordingly, $F_G$ will be given as:

$$F_G = 6 \times f \quad (3)$$

where q is:

$$q = Q_G/6 \quad (4)$$

$Q_G$: a quantity of the gas blown into the gas blow pipe Accordingly, $F_G$ will be given as:

$$F_G = k \times Q_G \times v \quad (5)$$

($Q_G \times v$ is represented by $f_G$ in Eq. (5)).

According to Fig. 4, it was found that if $f_G$ of each nozzle was identical, the volumetric oxygen transfer coefficient ($h^{-1}$) remained almost the same value. That was, if a force of the blow gas to depress the liquid level was identical, the same volumetric oxygen transfer coefficient was obtainable despite a varying nozzle bore. Further, it had been observed that the gas was drawn into the liquid when a $f_G$ value was 2.5 x $10^6$ [($cm^2$/s)$^2$] with each nozzle bore.

Consequently, if a relation between the volumetric oxygen transfer coefficient and a $f_G$ value is obtained beforehand by means of an arbitrary nozzle, then the volumetric oxygen transfer coefficient is obtainable by calculating a $f_G$ value without measuring a linear velocity at the liquid level even if a nozzle bore is changed.

## Example 3

In Example 2, in case of a single nozzle we obtained a $f_G$ value whereat the blow gas was about to be drawn into the liquid when a distance between the nozzle bore face and the still liquid level was changed.

The result is shown in Fig. 5. A distance between the nozzle bore face and the liquid level was specified at 10 mm, 20 mm and 40 mm.

It was found from Fig. 5 that a $f_G$ value whereat the gas was about to be drawn into the liquid was proportional to the distance between the nozzle bore face and the liquid level. Accordingly, if the distance between the nozzle bore face and the still liquid level is h cm, then a $f_G$ value whereat the gas is not drawn into the liquid may be adjusted to 2.5 x $10^5$ x h or below.

## Example 4

A culture test was carried out in the culture tank used in Example 1. Then, another culture test was carried out as a comparison example according to a conventional process.

JTC-1 (established cell line being suspensible and derived from ascitic hepatoma of a rat) was used as a cell subjected to the test. A mixture of 10% new-born calf serum and DM-160 (made by Kyokuto Seiyaku) was used as the culture medium. A quantity of the prepared culture medium was 2.5ℓ. The stirring blade of A type in Fig. 3 was used, and the nozzle blow face of the gas blow pipe was 20 mm away from the liquid level.

Culture conditions were as follows. The number of rotations of the stirring blade was kept constant at 80 rpm, and a culture temperature was controlled to 36 ± 1°C. The oxygen-containing gas was an air and carbon dioxide mixture, which was held at a saturation steam pressure by sparging it on a 37 °C water. It was then fed into the tank through a 0.45μ sterilizing filter. At the beginning of culture, the liquid was aerated with the mixed gas at a rate of 0.2 ℓ/min so as not to form a dimple on the level.

Then, an initial concentration of the carbon dioxide gas was 5V/V%. A formation of the dimple on the liquid level by the blown oxygen-containing gas was started when a dissolved oxygen concentration become 2 ppm or below. A flow rate of the oxygen-containing gas was controlled thereafter so as to keep a dissolved oxygen concentration at 2 ppm. A mixing ratio of the carbon dioxide is decreased after a cell density has reached 1.5 x $10^6$ cells/mℓ. Then in the comparison example, air mixed gas having carbon dioxide gas concentration of 5V/V% was aerated constantly at 0.2 ℓ/min. Further, the culture medium was replaced during the test for removal of wastes and feed of a nutritive source. The ratio of replacement was 100%.

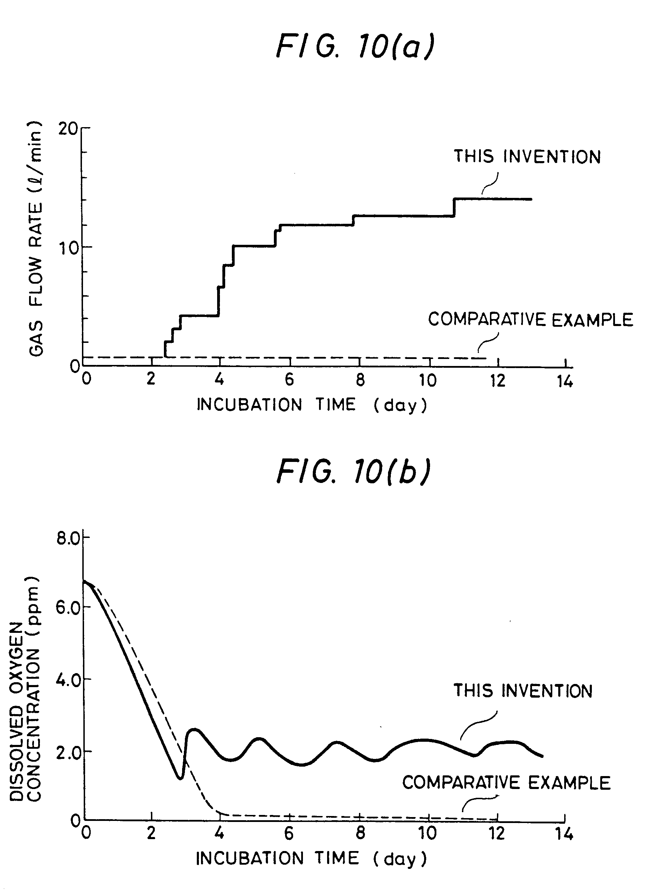

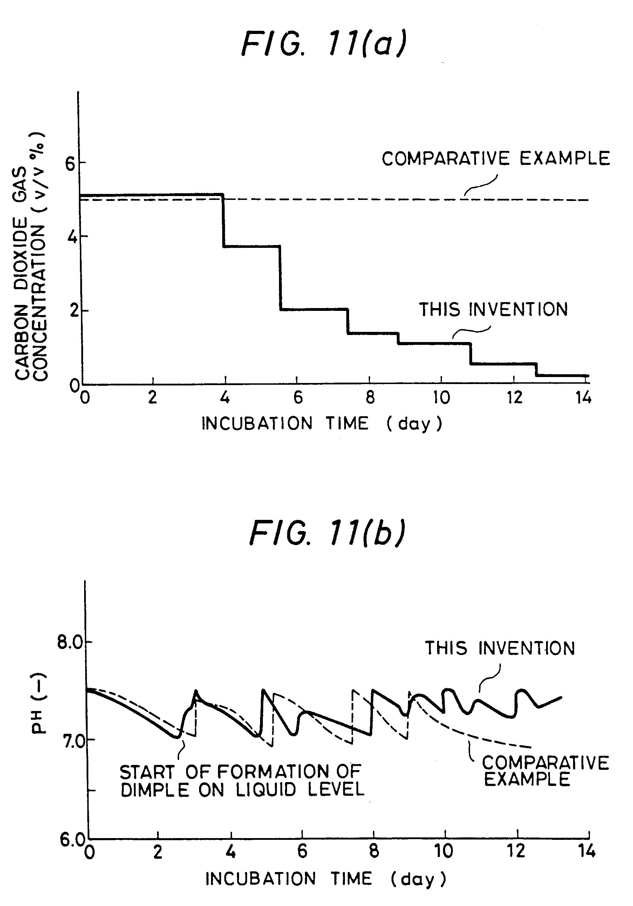

The culture results are shown in Fig. 9, Fig. 10 and Fig. 11. As shown in Fig. 9, while the cells reached the uppermost limit at a cell density of 3 x $10^6$ cells/mℓ or so in the comparison example (conventional process), the cells increased at the same propagation rate as in the comparison example and propagated almost exponentially up to 1.2 x $10^7$ cells/mℓ in the invention. Both pH-values throughout the culture period were within 6.9 to 7.5 as shown in Fig. 11, and thus a pH-value was not influential as a limiting factor to the growth. Accordingly, the aforementioned difference is due to the following reason. A dissolved oxygen density in the comparison example was almost zero at a cell density of $1.4 \times 10^6$ cells/mℓ or over as shown in Fig. 10, which provided a limiting factor to propagation. On the other hand, since a high oxygen transfer rate was obtainable as shown in Example 1 in the invention, a dissolved oxygen concentration could be kept at 1.8 to 2.6 ppm until the end of cultivation, and thus the oxygen necessary for propagation could be fed sufficiently.

Then, there was no significant foaming observed during the culture, and the cell viability was 85% at the end of the culture, which was not a significant decrease as compared with 88% at the time of culture start.

As described above, it has been confirmed that cells could be cultured at a density more than three times as high as with the conventional process even if air was used as an oxygen-containing gas according to the invention.

It is to be noted that a liquid phase and gas phase of the carbon dioxide can be exchanged in good condition according to the invention. This appears in the change of pH at a point in time when a dimple is about to be formed on the liquid level. While decreasing in the comparison example, a pH-value increased in the invention. That is, a mass transfer is accelerated of a gas between a liquid phase and a gas phase by blowing the oxygen containing gas to the liquid level and forming the dimple there, and thus the carbon dioxide in a liquid phase can be discharged to a gas phase in good condition in the invention. Further, even in a high density culture where production rate of carbon dioxide is high, the mass transfer rate of a gas between a liquid phase and a gas phase can be increased in the invention; therefore, the carbon dioxide can be discharged from a liquid phase to a gas phase by utilizing an oxygen-containing gas with a low carbon dioxide gas concentration. A decrease in pH-value resulting from an accumulation of the dissolved carbon dioxide can be suppressed accordingly as shown in Fig. 11.

According to the invention, a mass transfer of gas between a gas phase at the upper portion of a mechanical stirring culture tank and a liquid phase of a culture solution can be accelerated while foaming of the culture in the tank is suppressed.

## Claims

1. A method for culturing animal cells in a mechanically stirred culture tank, wherein a culture solution is agitated with the formation of turbulence or vortexes at the surface of the liquid being suppressed, an oxygen-containing gas is discharged continuously to the surface of the liquid from at least one blow nozzle, characterized in that the product ($f_g$) of the flow rate of the gas and the gas linear velocity at the nozzle tip does not exceed a value of $2.5 \times 10^5$ $((cm^2/s)^2)$ multiplied by the distance h (cm) from the blow nozzle tip to the still surface of the liquid, and that at least one dimple is formed by the gas.

2. The method of claim 1, wherein said gas is discharged so as to adjust a vertical linear velocity of the oxygen-containing gas discharged from the blow nozzle 1 mm in bore at the surface of the liquid from 5 m/s up to and including 65 m/s.

3. The method of claim 1, wherein the animal cells are dispersed with a stirrer and the culture solution is agitated while keeping the surface of the culture solution substantially smooth.

4. The method of claim 3, wherein said stirrer has a stirring blade having a center-punched rectangular plate twisted 1/2 turn.

5. The method of any of the preceding claims, wherein a culture solution is agitated while suppressing the formation of turbulence and vortexes at the surface of the liquid, and while generating a flow of the culture solution from the bottom portion of the dimple toward the surface of the liquid, and an oxygen-containing gas is dissolved into the culture solution.

6. The method of any of the preceding claims, wherein said oxygen-containing gas is discharged toward said surface of the liquid under conditions whereby an inverted conical dimple is formed on said surface of the liquid and oxygen is dissolved in said culture solution while substantially preventing bubbles from being drawn into said culture solution.

7. The method of claim 6, wherein the flow rate of said oxygen-containing gas is increased with time so as to provide a dissolved oxygen concentration above a value corresponding to that which would be a limiting factor for propagation of said animal cells.

8. The method of claim 7, wherein the oxygen content of said oxygen-containing gas is increased when said flow rate approximates to a level at which said product $f_G$ is $2.5 \times 10^5$ (-

(cm$^2$/s)$^2$) x h.

9. An apparatus for culturing animal cells having a culture tank (1) for storing a culture solution in which animal cells are dispersed, a stirrer (3) for agitating the culture solution, means (2) for feeding an oxygen-containing gas to the liquid surface of the culture solution, wherein at least one gas blow nozzle (2b) is provided above the surface of the liquid for discharging the gas continuously toward said surface, and control means for controlling the discharge pressure of the gas from the nozzle to form a dimple on the surface of the liquid.

10. The apparatus according to claim 9, wherein said gas blow nozzle (2b) discharges said oxygen-containing gas almost vertically toward the surface of the liquid.

11. The apparatus of claim 9, wherein said stirrer (3) has a stirring blade with a center-punched rectangular plate twisted 1/2 turn.

**Patentansprüche**

1. Verfahren zur Züchtung tierischer Zellen in einem mechanisch gerührten Züchtungsbehälter, wobei eine Züchtungslösung unter Vermeidung der Bildung von Turbulenzen oder Strudeln auf der Flüssigkeitsoberfläche gerührt wird und ein sauerstoffhaltiges Gas aus zumindest einer Blasdüse kontinuierlich auf die Flüssigkeitsoberfläche ausgeblasen wird, dadurch gekennzeichnet, daß das Produkt (f$_G$) der Gasdurchflußrate und der Gaslineargeschwindigkeit an der Düsenmündung den Wert 2,5 x 10$^5$ (-(cm$^2$/s)$^2$), multipliziert mit der Entfernung h (cm) von der Düsenmündung zur glatten Flüssigkeitsoberfläche, nicht überschreitet und daß zumindest eine Vertiefung durch das Gas gebildet wird.

2. Verfahren nach Anspruch 1, bei dem das Gas derartig ausgeblasen wird, daß die Gaslineargeschwindigkeit des sauerstoffhaltigen Gases, das von einer Blasdüse mit 1mm Bohrung auf die Flüssigkeitsoberfläche ausgeblasen wird, 5 m/s bis einschließlich 65 m/s beträgt.

3. Verfahren nach Anspruch 1, bei dem die tierischen Zellen mittels eines Rührers dispergiert werden und die Züchtungslösung umgerührt wird, während die Oberfläche der Züchtungslösung im wesentlichen glatt gehalten wird.

4. Verfahren nach Anspruch 3, bei dem der Rührer ein Rührblatt mit einer um eine halbe Drehung verwundenen, mittig ausgestanzten rechteckigen Platte aufweist.

5. Verfahren nach einem der vorhergehenden Ansprüche, bei dem eine Züchtungslösung unter Vermeidung der Bildung von Turbulenzen oder Strudeln auf der Flüssigkeitsoberfläche und unter Erzeugung einer Strömung von der Züchtungslösung aus dem Bodenabschnitt der Vertiefung in Richtung der Flüssigkeitsoberfläche gerührt wird, und ein sauerstoffhaltiges Gas in der Züchtungslösung gelöst wird.

6. Verfahren nach einem der vorhergehenden Ansprüche, bei dem das sauerstoffhaltige Gas unter derartigen Bedingungen in Richtung der Flüssigkeitsoberfläche ausgeblasen wird, daß eine invertierte, konische Vertiefung auf der Flüssigkeitsoberfläche gebildet wird und der Sauerstoff in der Züchtungslösung gelöst wird, während im wesentlichen verhindert wird, daß Blasen in die Züchtungslösung gezogen werden.

7. Verfahren nach Anspruch 6, wobei die Durchflußrate des sauerstoffhaltigen Gases mit der Zeit erhöht wird, um eine Konzentration gelösten Sauerstoffs zu erhalten, der oberhalb desjenigen Wertes liegt, welcher ein begrenzender Faktor für die Vermehrung der tierischen Zellen wäre.

8. Verfahren nach Anspruch 7, wobei der Sauerstoffgehalt des sauerstoffhaltigen Gases erhöht wird, wenn die Durchflußrate sich einem Wert nähert, bei dem das Produkt f$_G$ 2,5 x 10$^5$ (-(cm$^2$/s)$^2$) x h beträgt.

9. Vorrichtung zur Züchtung tierischer Zellen mit einem Züchtungsbehälter (1) zur Aufnahme einer Züchtungslösung, in der tierische Zellen dispergiert sind, mit einem Rührer (3) zum Umrühren der Züchtungslösung, mit einer Einrichtung (2) zur Zuführung eines sauerstoffhaltigen Gases auf die Flüssigkeitsoberfläche der Züchtungslösung, wobei zumindest eine Gasblasdüse (2b) oberhalb der Flüssigkeitsoberfläche vorgesehen ist, um das Gas kontinuierlich in Richtung der Oberfläche auszublasen, und mit einer Regeleinrichtung zur Regelung des Ausblasdrucks des Gases aus der Düse, um eine Vertiefung in der Flüssigkeitsoberfläche zu bilden.

10. Vorrichtung nach Anspruch 9, bei der die Gasblasdüse (2b) das sauerstoffhaltige Gas nahezu vertikal in Richtung der Flüssigkeitsoberfläche ausbläst.

**11.** Vorrichtung nach Anspruch 9, wobei der Rührer (3) ein Rührblatt mit einer um eine halbe Drehung verwundenen, mittig ausgestanzten rechteckigen Platte aufweist.

## Revendications

**1.** Procédé de culture de cellules animales dans un récipient de culture agité mécaniquement, dans lequel on agite une solution de culture en supprimant la formation de turbulences ou de tourbillons à la surface du liquide, on déverse un gaz contenant de l'oxygène en continu sur la surface du liquide à partir d'au moins une buse d'insufflation, caractérisé en ce que le produit ($f_G$) du débit du gaz et de la vitesse linéaire du gaz à l'extrémité de la buse ne dépasse pas une valeur de $2,5 \times 10^5$ $((cm^2/s)^2)$ multipliée par la distance h (cm) entre l'extrémité de la buse d'insufflation et la surface tranquille du liquide et qu'au moins une ride est formée par le gaz.

**2.** Procédé selon la revendication 1, dans lequel ledit gaz est déchargé de façon à assurer pour le gaz contenant de l'oxygène déversé à partir d'une buse d'insufflation de 1 mm de calibre sur la surface du liquide, une vitesse linéaire verticale de 5 m/s à 65 m/s au plus.

**3.** Procédé selon la revendication 1, dans lequel on disperse les cellules animales avec un agitateur et on agite la solution de culture tout en maintenant la surface de la solution de culture sensiblement lisse.

**4.** Procédé selon la revendication 3, dans lequel ledit agitateur a une lame d'agitation ayant une plaque rectangulaire poinçonnée en son centre et tordue d'1/2 tour.

**5.** Procédé selon l'une quelconque des revendications précédentes, dans lequel on agite une solution de culture tout en supprimant la formation de turbulences et de tourbillons à la surface du liquide, et tout en générant un flux de solution de culture à partir du fond de la ride vers la surface du liquide, et un gaz contenant de l'oxygène se dissout dans la solution de culture.

**6.** Procédé selon l'une quelconque des revendications précédentes, dans lequel on déverse ledit gaz contenant de l'oxygène vers ladite surface de liquide dans des conditions telles qu'il se forme une ride conique inversée à ladite surface du liquide et de l'oxygène se dissout dans ladite solution de culture, l'introduction de bulles dans ladite solution de culture étant sensiblement empêchée.

**7.** Procédé selon la revendication 6, dans lequel on augmente le débit dudit gaz contenant de l'oxygène avec le temps de manière à fournir une concentration d'oxygène dissous supérieure à une valeur correspondant à celle qui serait un facteur limitatif pour la propagation desdites cellules animales.

**8.** Procédé selon la revendication 7, dans lequel on augmente la teneur en oxygène dudit gaz contenant de l'oxygène lorsque ledit débit approche un niveau auquel ledit produit $f_G$ est de $2,5 \times 10^5$ $((cm^2/s)^2 \times h$.

**9.** Appareil pour cultiver des cellules animales ayant une cuve de culture (1) pour conserver une solution de culture dans laquelle des cellules animales sont dispersées, un agitateur (3) pour agiter la solution de culture, un moyen (2) pour alimenter un gaz contenant de l'oxygène à la surface du liquide de la solution de culture, où on dispose au moins une buse d'insufflation de gaz (2b) au-dessus de la surface du liquide pour déverser le gaz en continu vers ladite surface, et un moyen de commande pour régler la pression de sortie du gaz à partir de la buse pour former une ride à la surface du liquide.

**10.** Appareil selon la revendication 9, dans lequel ladite buse d'insufflation de gaz (2b) déverse ledit gaz contenant de l'oxygène presque verticalement vers la surface du liquide.

**11.** Appareil selon la revendication 9, dans lequel ledit agitateur (3) présente une lame d'agitation avec une plaque rectangulaire poinçonnée en son centre et tordue d'1/2 tour.

# FIG. 1

# FIG. 2

2a

2c

2b

A ◄――――――――――――――― A

2c

2a

2b

## FIG. 3(a)

## FIG. 3(b)

## FIG. 4

## FIG. 5

PORE DIAMETER OF NOZZLE
- ◒ --- 0.3 mmφ
- ◓ --- 0.4 mmφ
- ● --- 1.0 mmφ
- ◑ --- 2.5 mmφ
- ◐ --- 5.0 mmφ

AREA OF ENTRAPMENT OF BUBBLES

AREA OF NO ENTRAPMENT OF BUBBLES

$f_G$ ( $cm^4/sec^2$ )

DISTANCE BETWEEN NOZZLE BLOW SURFACE AND LIQUID LEVEL ( cm )

## FIG. 6

DIMPLE IS FORMED ON LIQUID LEVEL

ENTRAPMENT OF BUBBLES

VOLUMETRIC OXYGEN TRANSFER COEFFICIENT ( $h^{-1}$ )

LINEAR VELOCITY OF AIR JETTED FROM NOZZLE ON LIQUID LEVEL IN PERPENDICULAR DIRECTION (vm/sec )

14

## FIG. 7

## FIG. 8

# FIG. 9

## FIG. 10(a)

## FIG. 10(b)

## FIG. 11(a)

COMPARATIVE EXAMPLE

THIS INVENTION

CARBON DIOXIDE GAS CONCENTRATION ( v/v %)

INCUBATION TIME ( day )

## FIG. 11(b)

THIS INVENTION

PH (−)

START OF FORMATION OF DIMPLE ON LIQUID LEVEL

COMPARATIVE EXAMPLE

INCUBATION TIME ( day )

18